# EUROPEAN PATENT SPECIFICATION

(11) **EP 2 842 641 B1**
(45) Date of publication and mention of the grant of the patent: **09.10.2019**
(21) Application number: 13781836.5
(22) Date of filing: 26.04.2013
(51) Int. Cl.: B05B 17/06, A01M 1/20, A61L 2/18, A61L 2/22, A61L 9/14, F24F 6/12

(54) **ULTRASONIC ATOMIZATION DEVICE**
ULTRASCHALLZERSTÄUBER
DISPOSITIF D'ATOMISATION À ULTRASONS

(30) Priority: 27.04.2012 JP 2012103621
(43) Date of publication of application: 04.03.2015
(73) Proprietor: Sumitomo Chemical Company Limited, Tokyo 104-8260 (JP)
(72) Inventor: KAWANO, Hiroyuki, Takarazuka-shi Hyogo 665-8555 (JP); HARADA, Tetsuo, Takarazuka-shi Hyogo 665-8555 (JP); TAKAHATA, Daisuke, Ageo-shi Saitama 362-8561 (JP); UEDA, Kazuyuki, Ageo-shi Saitama 362-8561 (JP)
(74) Representative: Vossius & Partner Patentanwälte Rechtsanwälte mbB
(86) International application number: PCT/JP2013/062346
(87) International publication number: WO 2013/161986

(56) References cited:
- WO-A1-00/47335
- JP-A- S58 216 753
- JP-A- S58 216 753
- JP-A- 2002 536 173
- JP-A- 2004 147 643
- JP-A- 2012 115 828
- US-A1- 2009 242 663
- US-A1- 2011 315 786

## Description

### Technical Field

The present invention relates to an ultrasonic atomizing device for atomizing a solution by ultrasonic vibration.

### Background Art

An ultrasonic atomizing device as disclosed in document WO00/473335 has been known as means for atomizing, in an interior or exterior space, liquid such as a solution containing an active ingredient. The ultrasonic atomizing device includes (i) a piezoelectric element which generates ultrasonic vibration when supplied with electricity and (ii) a vibrating plate which has many micropores and is attached to the piezoelectric element. The ultrasonic atomizing device is configured to atomize liquid by (i) supplying the liquid to the micropores and (ii) causing ultrasonic vibration on the vibrating plate by vibration of the piezoelectric element.

An atomizing device of Patent Literature 1 includes a pressurizing chamber for containing a liquid, a nozzle plate having a plurality of nozzles and facing the pressurizing chamber, and an electric vibrator for urging the nozzle plate and exciting flexural vibration, and a protrusion section is provided in the nozzle plate and micropores are provided in the protrusion section. The micropores directly contact with a liquid and are directed in a horizontal direction (a direction vertical to a direction of gravity). With this, the atomizing device of Patent Literature 1 atomizes a liquid in the horizontal direction.

An atomizer of Patent Literature 2 includes a film capable of vibrating for atomizing a liquid and a vibration section for vibrating the film. The film has a first curving portion and a second curving portion having a different curvature from that of the first curving portion, and atomizes a liquid in contact with the film by vibration of the vibration section. The atomizer of Patent Literature 2 atomizes a liquid downward (in a direction of gravity).

In an ultrasonic wave atomizing device of Patent Literature 3, a piezoelectric element is vibrated by a complex constituting of the piezoelectric element and a vibrator is driven and such vibration is propagated to the vibrator. A liquid supplied to a bottom surface of a vibrator in contact with a liquid holding agent is atomized through pores provided in the vibrator in accordance with the vibration of the vibrator.

In a piezoelectric atomizer of Patent Literature 4, a solution having viscosity of 5 mPa·s (at 20 °C) to 30 mPa·s (at 20 °C) is used.

### Citation List

### Patent Literatures

Patent Literature 1
   Japanese Patent Application Publication, *Tokukaisho*, No. 58-216753 (Publication date: December 16, 1983)
Patent Literature 2
   U.S. Pat. No. 7472701 (Registration date: January 6, 2009)
Patent Literature 3
   Japanese Patent Application Publication, *Tokukaihei*, No. 6-7721 (Publication date: January 18, 1994)
Patent Literature 4
   Japanese Patent Application Publication, *Tokukai*, 2009-269893 (Publication date: November 19, 2009)

### Summary of Invention

### Technical Problem

However, techniques of Patent Literatures 1 to 4 have the following problems.

Specifically, a liquid directly contacts with a vibrating plate in the atomizing device of Patent Literature 1 and in the atomizer of Patent Literature 2, and therefore, if an atomizing opening is provided upward, the liquid becomes further from the vibrating plate as a residual liquid is reduced, and consequently atomization of the liquid to an outside of the device is hindered. In addition, when the residual liquid is reduced, air bubbles increase between the vibrating plate and a surface of the liquid, and consequently the atomization of the liquid to the outside of the device is hindered.

In the atomizing device of Patent Literature 1, the atomizing opening is directed horizontally, the surface of the liquid becomes lower as the residual liquid is reduced. Therefore, in the atomizing device of the Patent Literature 1, when the surface of the liquid becomes lower than the nozzle, the atomization of the liquid to the outside of the device is hindered.

In the atomizer of Patent Literature 2, the liquid is atomized downward, so that it is possible to atomize a whole amount of the liquid, however, a weight of the liquid is applied as a load to the vibrating plate. Therefore, there is a problem in that the vibrating plate is degraded in a shorter time.

As described above, in both the atomizing device of Patent Literature 1 and the atomizer of Patent Literature 2, it is difficult to atomize the liquid to the outside of the device stably and sustainably.

In the ultrasonic wave atomizing device of Patent Literature 3, a liquid which has been permeated to the liquid holding agent is atomized upward (in a direction opposite from that of gravity) via the vibrator which is in contact with the liquid holding agent. However, in the ultrasonic wave atomizing device of the Patent Literature 3, it is difficult to obtain an enough atomizing height because a center of the vibrator is curved. Therefore, because diffusibility of the liquid to be atomized cannot be satisfactorily obtained, it is difficult to widely spread an effect (sterilization, killing insects, etc.) obtained by atomizing the liquid within a wide range.

In the piezoelectric atomizer of Patent Literature 4, because a solution having viscosity of 5 mPa·s (at 20 °C) to 30 mPa·s (at 20 °C) is used and a vibrator is flat, an atomizing amount of the solution, atomizing stability, etc. are not enough.

The present invention has been made in view of the above problems, and an object of the present invention is to provide an ultrasonic wave atomizing device capable of improving diffusibility of an atomized solution.

### Solution to Problem

In order to achieve the above object, an ultrasonic wave atomizing device in accordance with the present invention includes: a liquid absorbent wick for absorbing a solution from a solution storage container; and a vibrating plate which has multiple micropores penetrating the vibrating plate in a thickness direction and atomizes the solution by vibration of a piezoelectric vibrator which generates ultrasonic vibration when supplied with electricity, the vibrating plate having a convex part which has a frustum shape and is supplied with the solution via the liquid absorbent wick, the solution being a solution in which at least an organic solvent and an active ingredient are mixed and whose viscosity is 1.7 mPa·s (at 20 °C) to 5.0 mPa·s (at 20 °C).

According to the above structure, an ultrasonic wave atomizing device according to the present invention includes a liquid absorbent wick for absorbing a solution from a solution storage container. Therefore, by immersing one end of the liquid absorbent wick in a solution in the solution storage container and directing the other of the liquid absorbent wick upward (in a direction opposite from that of gravity), it is possible to atomize the solution upward by vibrating the vibrating plate.

In the ultrasonic wave atomizing device according to the present invention, the vibrating plate has the frustum-shaped convex part to which the solution is supplied through the liquid absorbent wick. Therefore, the ultrasonic wave atomizing device according to the present invention can have a high atomizing height of the solution, as compared with a case where the solution is atomized with use of a vibrating plate having a conventional dome shape, plate-like shape, or the like. Therefore, the ultrasonic wave atomizing device according to the present invention can improve diffusibility of the solution around the ultrasonic wave atomizing device, and it is possible to widely spread an effect brought about by an active ingredient.

In the ultrasonic wave atomizing device in accordance with the present invention, there is used a solution in which at least an organic solvent and an active ingredient are mixed and which has viscosity of 1.7 mPa·s (at 20 °C) to 5.0 mPa·s (at 20 °C). With this configuration, the ultrasonic wave atomizing device in accordance with the present invention improves diffusibility of the solution with spontaneous evaporativity caused by the organic solvent. In addition, by combining a vibrating plate having a frustum-shaped convex part with a range of viscosity of the solution, an atomizing amount, an atomizing height, etc. of the solution are improved. This makes it possible to improve the diffusibility of the solution.

In order to achieve the above object, an ultrasonic wave atomizing device according to the present invention includes: a liquid absorbent wick for absorbing a liquid to be atomized; a piezoelectric element which has a ring shape when seen from a plan view and generates ultrasonic vibration in a radial direction when supplied with electricity; and a vibrating plate which is fixed to the piezoelectric element so that the vibrating plate may cover a center opening of the piezoelectric element, the vibrating plate having a center being substantially concentric with the center opening of the piezoelectric element, having a truncated-cone-shaped convex part protruding in a direction in which the liquid is atomized, and having multiple micropores in at least an upper base of the truncated-cone-shaped convex part, the multiple micropores penetrating the vibrating plate in a thickness direction, the liquid being supplied to the vibrating plate via the liquid absorbent wick.

According to the above structure, in the ultrasonic wave atomizing device according to the present invention, the vibrating plate has the truncated-cone-shaped convex part to which the liquid is supplied through the liquid absorbent wick. Therefore, the ultrasonic wave atomizing device according to the present invention can have a height at which the liquid is atomized higher, as compared with a case where a liquid is atomized with use of a vibrating plate having a conventional dome shape or the like. Therefore, the ultrasonic wave atomizing device according to the present invention can improve diffusibility of the liquid around the ultrasonic wave atomizing device, and, in a case where the liquid is, for example, an insecticide, it is possible to widely spread an effect of killing insects.

Because the ultrasonic wave atomizing device according to the present invention includes the vibrating plate having the truncated-cone-shaped convex part, the ultrasonic wave atomizing device has higher durability than conventional vibrating plates. Therefore, the ultrasonic wave atomizing device according to the present invention can reduce burdens on a user, such as time, effort, and costs for exchanging a vibrating plate.

### Advantageous Effects of Invention

As described above, an ultrasonic wave atomizing device in accordance with the present invention includes: a liquid absorbent wick for absorbing a solution from a solution storage container; and a vibrating plate which has multiple micropores penetrating the vibrating plate in a thickness direction and atomizes the solution by vibration of a piezoelectric vibrator which generates ultrasonic vibration when supplied with electricity, the vibrating plate having a convex part which has a frustum shape and is supplied with the solution via the liquid absorbent wick, the solution being a solution in which at least an organic solvent and an active ingredient are mixed and whose viscosity is 1.7 mPa·s (at 20 °C) to 5.0 mPa·s (at 20 °C).

Further, as described above, an ultrasonic wave atomizing device according to the present invention includes: a liquid absorbent wick for absorbing a liquid to be atomized; a piezoelectric element which has a ring shape when seen from a plan view and generates ultrasonic vibration in a radial direction when supplied with electricity; and a vibrating plate which is fixed to the piezoelectric element so that the vibrating plate may cover a center opening of the piezoelectric element, the vibrating plate having a center being substantially concentric with the center opening of the piezoelectric element, having a truncated-cone-shaped convex part protruding in a direction in which the liquid is atomized, and having multiple micropores in at least an upper base of the truncated-cone-shaped convex part, the multiple micropores penetrating the vibrating plate in a thickness direction, the liquid being supplied to the vibrating plate via the liquid absorbent wick.

Therefore, an ultrasonic wave atomizing device according to the present invention can improve diffusibility of an atomized liquid.

### Brief Description of Drawings

Fig. 1 is a view schematically illustrating an ultrasonic wave atomizing device according to this embodiment.
Fig. 2 is an enlarged view illustrating an atomizing section of an ultrasonic wave atomizing device according to this embodiment.
Fig. 3 is views schematically illustrating a vibrating plate according to this embodiment, and (a) of Fig. 3 is a top view and (b) of Fig. 3 is a cross-sectional view.
Fig. 4 is a view illustrating a modified example of an ultrasonic wave atomizing device according to this embodiment.
Fig. 5 is a view schematically illustrating another vibrating plate according to this embodiment, and (a) of Fig. 5 is a top view and (b) of Fig. 5 is a cross-sectional view.
Fig. 6 is a view schematically illustrating still another vibrating plate according to this embodiment, and (a) of Fig. 6 is a top view and (b) of Fig. 6 is a cross-sectional view.
Fig. 7 is an enlarged view illustrating another atomizing section of an ultrasonic wave atomizing device according to this embodiment.
Fig. 8 is a table showing relationships between viscosity and atomizing amount etc. of solution.
Fig. 9 is a view showing relationships between viscosity and atomizing amount, which are obtained when a plate-like, dome-shaped, and truncated-cone-shaped vibrating plates are used.

### Description of Embodiments

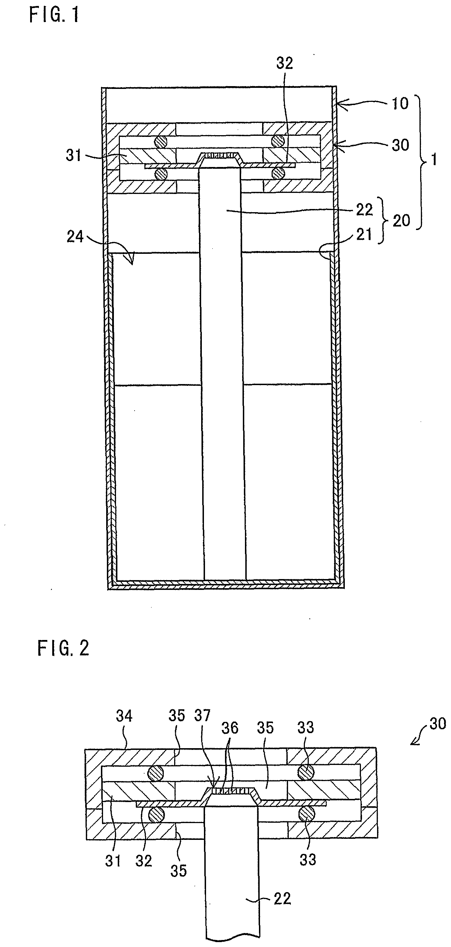

First, the following description discusses, with reference to Fig. 1 etc., an ultrasonic atomizing device 1 in accordance with this embodiment. Fig. 1 is a view schematically illustrating the ultrasonic atomizing device 1. Fig. 2 is an enlarged view illustrating an atomizing section 30 of the ultrasonic atomizing device 1.

### (Ultrasonic atomizing device 1)

The ultrasonic atomizing device 1 is a device for atomizing a solution by ultrasonic vibration. The ultrasonic atomizing device 1 includes (i) a device body 10 which includes the atomizing section 30 and (ii) a solution container (solution storage container) 20 which is housed in the device body 10. As the solution, a solution to be used as an insecticide, a pesticide, a fragrance, or the like is used.

### (Device body 10)

The device body 10 houses the solution container 20 therein and includes the atomizing section 30. The solution container 20 may be removably housed in the device body 10. As shown in Fig. 2, the atomizing section 30 includes (i) a piezoelectric element 31 which generates ultrasonic vibration when supplied with electricity, (ii) a vibrating plate 32 which atomizes a solution by vibration of the piezoelectric element 31, (iii) a couple of elastic rings 33 which are elastic annular members provided along a top surface of the piezoelectric element 31 and a bottom surface of the vibrating plate 32, respectively, and (iv) a casing 34 which holds the piezoelectric element 31 and the vibrating plate 32 by elastically sandwiching the piezoelectric element 31 and the vibrating plate 32 via the couple of elastic rings 33.

The piezoelectric element 31 is constituted by a thin circular piezoelectric ceramic plate which has an opening 35 at its center. The piezoelectric element 31 is polarized along its thickness direction, and generates ultrasonic vibration in a radial direction upon application of a high frequency voltage to electrodes (not illustrated) provided on both surfaces of the piezoelectric element 31. The piezoelectric element 31 is not limited provided that, for example, its thickness is 0.1 mm to 4.0 mm, its outer diameter is 6 mm to 60 mm. The piezoelectric element 31 has a ring shape when seen from a plan view, and has an opening in its center. The piezoelectric element 31 may have an oscillatory frequency of 30 kHz to 500 kHz.

The vibrating plate 32 is constituted by a thin circular plate made of, for example, nickel, nickel alloy, or ferroalloy. The vibrating plate 32 covers the opening 35 of the piezoelectric element 31, and, in Fig. 1, is joined (fastened) to a bottom surface of the piezoelectric element 31 so as to be concentric or substantially concentric (to substantially match) with the piezoelectric element 31. A thickness of the vibrating plate 32 is, for example, 0.02 mm to 2.0 mm, and an outer diameter of the vibrating plate 32 is, for example, 6 mm to 60 mm. The outer diameter of the vibrating plate 32 is selected as appropriate depending on the size of the piezoelectric element 31 so as to be larger than the inner diameter of the opening 35 of the piezoelectric element 31.

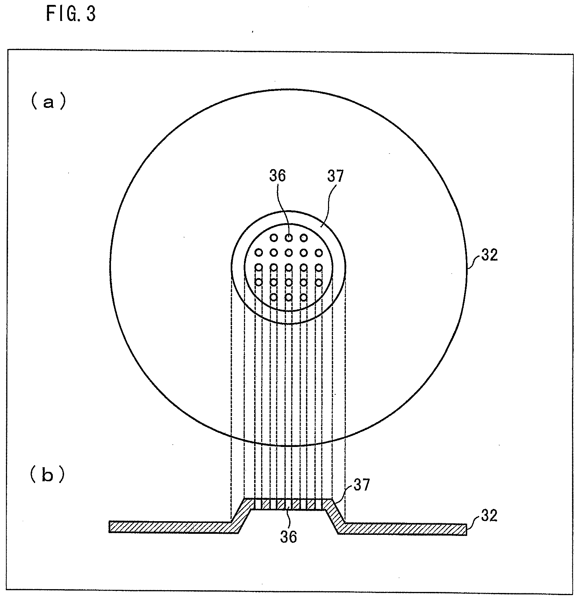

The vibrating plate 32 has, in its part that faces the opening 35 of the piezoelectric element 31, many micropores 36 passing through the vibrating plate 32 in a thickness direction. The diameter of each of the micropores 36 is preferably 3 pm to 150 µm. The vibrating plate will be described later with reference to Fig. 3 etc.

Note that, in Fig. 1, Fig. 2, etc. described later, the micropores 36 are provided only in an upper base of the convex part 37 of the vibrating plate 32. However, in the vibrating plate according to this embodiment, the micropores 36 may be provided over a whole surface of the vibrating plate.

In the ultrasonic atomizing device 1, a solution is supplied to the micropores 36, the piezoelectric element 31 generates ultrasonic vibration when supplied with electricity, and ultrasonic vibration generated in the vibrating plate 32 by vibration of the piezoelectric element allows the solution to be atomized.

There is provided the couple of elastic rings 33. The couple of elastic rings 33 are in contact with the top surface of the piezoelectric element 31 and the bottom surface of the vibrating plate 32, respectively, so as to be concentric with the piezoelectric element 31 and the vibrating plate 32, respectively. Here, the couple of elastic rings 33 are in a state of elastic deformation between the casing 34 and the top surface of the piezoelectric element 31 and between the casing 34 and the bottom surface of the vibrating plate 32, respectively.

Each of the couple of elastic rings 33 is preferably an O-ring having a section diameter of 0.5 mm to 3 mm. Further, the hardness of the couple of elastic rings 33 is preferably 20 IRHD to 90 IRHD. Such a couple of elastic rings 33 makes it possible to hold the piezoelectric element 31 and the vibrating plate 32 with appropriate elasticity, and to hold the piezoelectric element 31 at a prescribed position within the casing 34 without restricting vibration of the piezoelectric element 31. Accordingly, it is possible to atomize a solution in a more stable manner.

It should be noted that an elastic ring 33 in contact with the top surface of the piezoelectric element 31 and an elastic ring 33 in contact with the bottom surface of the vibrating plate 32 are preferably the same in terms of mean diameter [(Inner diameter + Outer diameter) / 2], section diameter, and hardness etc. In particular, the couple of elastic rings 33 preferably have the same mean diameter.

The couple of elastic rings 33 are made from, for example, nitrile rubber, fluororubber, ethylene propylene rubber, silicone rubber, acrylic rubber, hydrogenated nitrile rubber, and/or the like.

Each of the couple of elastic rings 33 can be, instead of the O-ring, a ring that has, for example, an elliptic, rectangular, triangular, or rhombic cross section. Alternatively, each of the couple of elastic rings 33 can be a ring that has, for example, a D-shaped, X-shaped, or T-shaped cross section. Each of the couple of elastic rings 33 does not necessarily have to be a circumferentially continuous and complete ring, and therefore can have a slit in a circumferential direction or have several slits intermittently along the circumferential direction.

Although the above description has discussed the vibrating plate 32 in the form of a thin circular plate which completely covers the opening 35 of the piezoelectric element 31, it is also possible to employ a vibrating plate in the form of a thin rectangular plate (i) which traverses the opening 35 of the piezoelectric element 31 and (ii) whose both ends are fastened to one surface of the piezoelectric element 31.

The atomizing section 30 is not limited to the above configuration, and can be constituted by a known piezoelectric atomizing section. The atomizing section 30 can be selected as appropriate.

### (Solution container 20)

The solution container 20 is constituted by a container body 21 and a liquid absorbent wick 22, and is removably housed in the device body 10.

The container body 21 is constituted by, for example, a cylindrical container which has a bottom surface and has an opening 24 at the top. The container body 21 contains a solution. The container body 21 is made from, for example, glass or synthetic resin.

The liquid absorbent wick 22 is, for example, made of nonwoven fabric and in columnar shape having a diameter of 2 mm to 6 mm. A lower portion of the liquid absorbent wick 22 is immersed in the solution contained in the container body 21. This makes it possible to supply the solution to an upper portion of the liquid absorbent wick 22 by capillary action.

The shape of the liquid absorbent wick 22 is not limited to a circular column, and can be a square column. The shape of the liquid absorbent wick 22 can be any shape. Furthermore, the thickness of the liquid absorbent wick 22 may be the thickness which allows the liquid absorbent wick 22 to pass through the opening 35 of the piezoelectric element 31 or through the inside of the convex part 37 of the vibrating plate 32.

The liquid absorbent wick 22 is preferably made of, for example, a porous material having continuous holes, a resin article having continuous cells, or an aggregation of resin fibers. Specific examples of materials from which the liquid absorbent wick 22 is made include, but not limited to: a resin article having continuous cells made of polyurethane, polyethylene, polyethylene terephthalate, polyvinyl formal, and polystyrene etc.; porous materials obtained by sintering of fine resin particles made mainly of polyethylene, polypropylene, and nylon, etc.; porous materials made of polyethylene fluoride etc.; aggregations of resin fibers, such as felt made of polyester, polypropylene, nylon, acrylic, rayon, wool, etc., and nonwoven fabric made of polyolefin fibers, polyester fibers, nylon fibers, rayon fibers, acrylic fibers, vinylon fibers, polychlal fibers, aramid fibers etc.; and porous sintered inorganic materials obtained by sintering of mainly inorganic powder such as ceramics. The specific examples of the materials further include the above materials treated with a surfactant.

How to house the solution container 20 in the device body 10 is not particularly limited, provided that (i) the solution container 20 is removably housed in the device body 10 and, (ii) while the device body 10 houses the solution container 20 therein, the liquid absorbent wick 22 is near or in contact with the convex part 37 (described later) of the vibrating plate 32. For example, the solution container 20 can be housed in the device body 10 by (i) being slidingly fitted into the device body 10 by being slid laterally or (ii) being rotatingly fitted into the device body 10 by being rotated laterally with a slight rotational angle.

### (Solution)

In the ultrasonic atomizing device 1, there is used a solution in which an organic solvent and an active ingredient are mixed and which has viscosity of 1.7 mPa·s (at 20 °C) to 5.0 mPa·s (at 20 °C). The solution is normally obtained by solving an active ingredient in an organic solvent.

As active ingredients, components for killing insects, sterilizing germs, fragrance, etc. are used. For example,
(1) synthetic pyrethroid compounds such as
   acrinathrin, allethrin, beta-cyfluthrin, bifenthrin, cycloprothrin, cyfluthrin, cyhalothrin, cypermethrin, empenthrin, deltamethrin, esfenvalerate, ethofenprox, fenpropathrin, fenvalerate, flucythrinate, flufenoprox, flumethrin, fluvalinate, halfenprox, imiprothrin, permethrin, prallethrin, pyrethrins, resmethrin, sigma-cypermethrin, silafluofen, tefluthrin, tralomethrin, transfluthrin, tetramethrin, phenothrin, cyphenothrin, alpha-cypermethrin, zeta-cypermethrin, lambda-cyhalothrin, gamma-cyhalothrin, furamethrin, tau-fluvalinate, metofluthrin, mepafurusurin, d-tefluthrin, dimefluthrin, 2,3,5,6-tetrafluoro-4-methylbenzyl=2,2-dimethyl-3-(1-propen yl)cyclopropane carboxylate, 2,3,5,6-tetrafluoro-4-(methoxymethyl)benzyl=2,2-dimethyl-3-(2-methyl-1-propenyl)cyclopropane carboxylate, and 2,3,5,6-tetrafluoro-4-(methoxymethyl)benzyl=2,2,3,3-tetrame thyl-cyclopropane carboxylate;
(2) organic phosphorus compounds such as
   acephate, aluminium phosphide, butathiofos, cadusafos, chlorethoxyfos, chlorfenvinphos, chlorpyrifos, chlorpyrifos-methyl, cyanophos:CYAP, diazinon, DCIP (dichlorodiisopropyl ether, dichlofenthion:ECP, dichlorvos:DDVP, dimethoate, dimethylvinphos, disulfoton, EPN, ethion, ethoprophos, etrimfos, fenthion:MPP, fenitrothion:MEP, fosthiazate, formothion, hydrogen phosphide, isofenphos, isoxathion, malathion, mesulfenfos, methidathion:DMTP, monocrotophos, naled:BRP, oxydeprofos:ESP, parathion, phosalone, phosmet:PMP, pirimiphos-methyl, pyridafenthion, quinalphos, phenthoate:PAP, profenofos, propaphos, prothiofos, pyraclorfos, salithion, sulprofos, tebupirimfos, temephos, tetrachlorvinphos, terbufos, thiometon, trichlorphon:DEP, vamidothion, phorate, and cadusafos;
(3) carbamate compounds such as
   alanycarb, bendiocarb, benfuracarb, BPMC, carbaryl, carbofuran, carbosulfan, cloethocarb, ethiofencarb, fenobucarb, fenothiocarb, fenoxycarb, furathiocarb, isoprocarb:MIPC, metolcarb, methomyl, methiocarb, NAC, oxamyl, pirimicarb, propoxur:PHC, XMC, thiodicarb, xylylcarb, and aldicarb;
(4) nereistoxin compounds such as
   cartap, bensultap, thiocyclam, monosultap, and bisultap;
(5) neonicotinoid compounds such as
   imidacloprid, nitenpyram, acetamiprid, thiamethoxam, thiacloprid, dinotefuran, and clothianidin;
(6) benzoyl urea compounds such as
   chlorfluazuron, bistrifluron, diafenthiuron, diflubenzuron, fluazuron, flucycloxuron, flufenoxuron, hexaflumuron, lufenuron, novaluron, noviflumuron, teflubenzuron, triflumuron, and triazuron;
(7) phenyl pyrazole compounds such as
   acetoprole, ethiprole, fipronil, vaniliprole, pyriprole, and pyrafluprole;
(8) Bt toxin insecticides such as
   raw spores and crystal toxin production derived from Bacillus thuringiensis bacteria and mixtures thereof;
(9) hydrazine compounds such as
   chromafenozide, halofenozide, methoxyfenozide, and tebufenozide;
(10) organic chlorine compounds such as
   aldrin, dieldrin, dienochlor, endosulfan, and methoxychlor;
(11) natural insecticide machine oil such as
   machine oil, nicotine-sulfate;
(12) other killing insecticides such as
   avermectin-B, bromopropylate, buprofezin, chlorphenapyr, cyromazine, D-D(1,3-Dichloropropene, emamectin-benzoate, fenazaquin, flupyrazofos, hydroprene, methoprene, indoxacarb, metoxadiazone, milbemycin-A, pymetrozine, pyridalyl, pyriproxyfen, spinosad, sulfluramid, tolfenpyrad, triazamate, flubendiamide, lepimectin, arsenic acid, benclothiaz, calcium cyanamide, calcium polysulfide, chlordane, DDT, DSP, flufenerim, flonicamid, flurimfen, formetanate, metam-ammonium, metam-sodium, methyl bromide, potassium oleate, protrifenbute, spiromesifen, sulfur, metaflumizone, spirotetramat, pyrifluquinazone, spinetoram, chlorantraniliprole, and tralopyril;
(13) Other repellents such as
   N,N-diethyl-m-toluamide, limonene, linalool, citronellal, menthol, menthone, hinokitiol, geraniol, eucalyptol, indoxacarb, Curran-3,4-diol, MGK-R-326, MGK-R-874, and BAY-KBR-3023;
(14) synergists such as
   5 - [2 - (2 - butoxyethoxy) ethoxymethyl]-6-propyl-1,3-benzodioxole, N-(2-ethylhexyl)bicyclo[2.2.1]hept-5-ene-2,3-dicarboximide, octachlorodipropylether, thiocyano isobornyl acetate, N-(2-ethylhexyl)-1-isopropyl-4-methylbicyclo[2.2.2]oct-5-ene-2,3-dicarboximide, but the present invention is not intended to be limited to examples in accordance with the present invention. Among them, in view of a component which is easily volatilized and is suitably used for pest control, metofluthrin, profluthrin, transfluthrin, mepafurusurin, d-tefurumesurin, and dimefluthrin are preferable, and metofluthrin is more preferable. Those components may be used solely or two or more components may be mixed. An active ingredient content in a solution is not particularly limited, and is, for example, 0.05 wt% to 10 wt%.

As the organic solvent, an organic solvent is used so that viscosity of a solution to be obtained by dissolving an active ingredient in the organic solvent may be 1.7 mPa·s (at 20 °C) to 5.0 mPa·s (at 20 °C). Examples of the organic solvent encompass: hydrocarbons such as n-paraffin (C₁₂-C₁₅) (cactus normal paraffin: JX Nippon Oil & Energy Corporation) and naphthenic solvents (Exxsol D110: Exxon Mobil Corporation); esters such as methyl laurate, coconut oil fatty acid methyl, isopropyl myristate, methyl oleate; alcohols such as 2-methyl-1-propanol; and glycol ethers such as propylene glycol monomethyl ether. Those may be used solely or two or more organic solvents may be mixed.

Note that an active agent, an antioxidant, etc. may be added as appropriate as long as the viscosity of the solution falls within the above viscosity range.

### (Modified Example of supply of solution to vibrating plate)

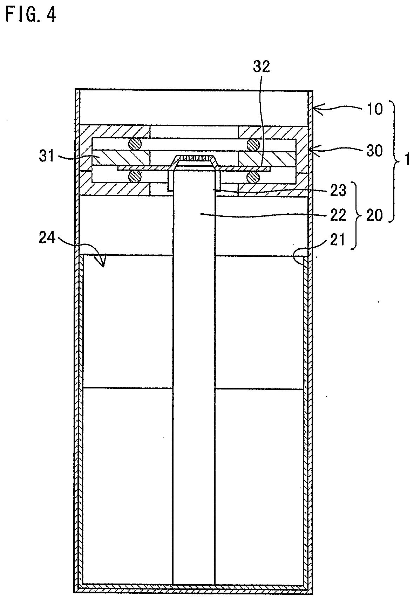

Supply of the solution to the vibrating plate 32 may be performed as follows, and this will be described with reference to Fig. 4. Fig. 4 is a view illustrating a modified example of the ultrasonic wave atomizing device 1. Note that the matters which have been described already with reference to Fig. 1 etc. will be omitted.

As shown in Fig. 4, the solution container 20 includes the container body 21, the liquid absorbent wick 22, and the absorber 23, and is removably provided to the device body 10.

The liquid absorbent wick 22 is, for example, made of nonwoven fabric and in columnar shape having a diameter of 2 mm to 6 mm. A lower portion of the liquid absorbent wick 22 is immersed in the solution contained in the container body 21. This makes it possible to supply the solution to an upper portion of the liquid absorbent wick 22 by capillary action. The absorber 23 is provided to the upper portion of the liquid absorbent wick 22.

The absorber 23 is provided to the upper portion of the liquid absorbent wick 22 so as to be integral with the liquid absorbent wick 22. That is, when the solution container 20 is provided to or removed from the ultrasonic atomizing device 1, the absorber 23 is also provided to or removed from the ultrasonic atomizing device 1 together with the solution container 20. The absorber 23 lies near or is in contact with the convex part 37 of the vibrating plate 32, and supplies, to the convex part 37, the solution absorbed by the liquid absorbent wick 22. A material of the absorber 23 may be the same as that of the liquid absorbent wick 22.

Note that, in this embodiment, the term "integral" means (i) members constitute a single member, (ii) members are assembled together, or (iii) the like.

The absorbent wick 22 and the absorber 23 are fixed to the container body 21, and removably attached to the solution container 20 (or the container body 21).

According to the above structure, in a case where the solution container 20 is detached from the ultrasonic atomizing device 1, the absorber 23 is detached together with the solution container 20 from the ultrasonic atomizing device 1 to an outside thereof, and does not remain in the ultrasonic atomizing device 1. Therefore, in a case where the solution in the solution container 20 is exhausted and the absorber 23 is dried, the absorber 23 as well as the solution container 20 is exchanged when the solution container 20 is exchanged, so that, when the ultrasonic atomizing device 1 is operated again, it is possible to prevent fibers etc. derived from the absorber 23 from clogging micropores of the vibrating plate 32. This effect is exerted particularly when the vibrating plate 32 is fixed to the side of the ultrasonic atomizing device 1.

Therefore, the ultrasonic atomizing device 1 can reduce the following situations: an atomizing amount of the solution is unstable because of this clogging; and a user is forced to exchange a high-cost vibrating plate.

The liquid absorbent wick 22 and/or the absorber 23 may be structured to be fixed to the side of the ultrasonic atomizing device 1.

### (Vibrating plate)

Next, the following description will discuss the vibrating plate 32 in detail with reference to Fig. 3. Fig. 3 is views schematically illustrating the vibrating plate 32, and (a) of Fig. 3 is a top view and (b) of Fig. 3 is a cross-sectional view.

As described above, the vibrating plate 32 is a circular thin plate made from, for example, nickel, and is joined (fastened) to the bottom surface of the piezoelectric element 31 so as to be concentric or to be substantially concentric (to substantially match) with the piezoelectric element 31. The vibrating plate 32 includes a convex part 37 protruding to have a truncated-cone shape, and the convex part 37 is provided so that the truncated-cone shape may be concentric with the piezoelectric element 31. As shown in (a) of Fig. 3 and (b) of Fig. 3, the plurality of micropores 36 are provided only in an upper base of the convex part 37.

Here, a frustum refers to a solid figure which is obtained by removing, from a cone or pyramid, a cone or pyramid which has the same apex and is reduced in size similarly. In other words, the frustum is a solid figure surrounded by a conical/pyramid surface and two parallel planes. In this embodiment, a frustum having a cone shape is referred to as "truncated cone", a frustum having a pyramid is referred to as "truncated pyramid", and a frustum having an n-gonal pyramid is referred to as "n-gonal truncated pyramid".

As described above, the convex part 37 has a truncated-cone shape when an upper surface on which the plurality of micropores 36 are provided is considered as the upper base and a rising surface of the convex part 37 in the vibrating plate 32 is considered as the conical/pyramid surface. In addition, the liquid absorbent wick 22 and the absorber 23 are located in a nonexistent part corresponding to a lower base, and the liquid is supplied to the convex part 37 from the absorber 23.

More specifically, the following description will discuss a case where a frustum has a truncated-cone shape. The upper base of the truncated-cone-shaped convex part 37 preferably has a diameter smaller than that of the cylindrical liquid absorbent wick 22. Although not existed, the lower base of the convex part 37 preferably has a diameter which is the same as or slightly larger than that of the liquid absorbent wick 22. In addition, the upper base of the truncated-cone-shaped convex part 37 preferably has a diameter of 1.0 mm or more but 7.0 mm or less. The lower base of the convex part 37 preferably has a diameter of 2.2 mm or more but 11.0 mm or less. The convex part 37 preferably has a height (distance between upper base and lower base) of 0.1 mm or more but 2.0 mm or less. An angle between the lower base of the convex part 37 and a slanting surface of the convex part 37 is preferably 45° or less.

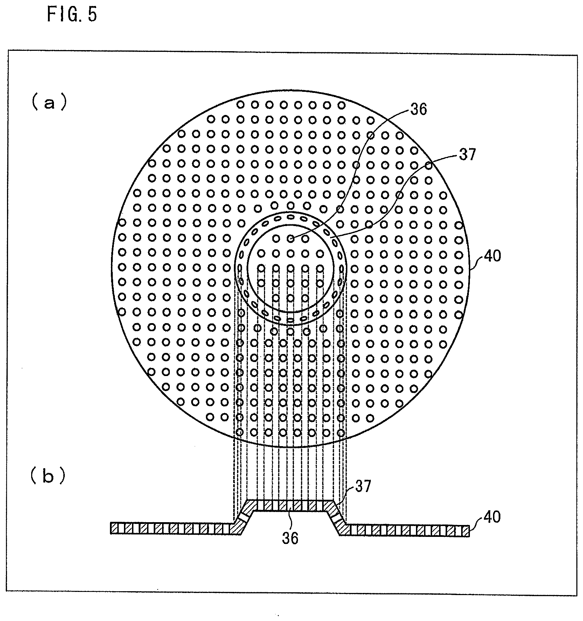

Fig. 5 illustrates a modification example of a vibrating plate according to this embodiment. Fig. 5 is a view schematically illustrating a vibrating plate 40, and (a) of Fig. 5 is a top view and (b) of Fig. 5 is a cross-sectional view.

The vibrating plate 40 is different from the vibrating plate 32 in the following point. That is, in the vibrating plate 32, the micropores 36 are provided only in the upper base of the convex part 37 of the vibrating plate 32, however, in the vibrating plate 40, the micropores 36 are provided over the whole vibrating plate 40. In this embodiment, not only the vibrating plate 32 but also the vibrating plate 40 can be used.

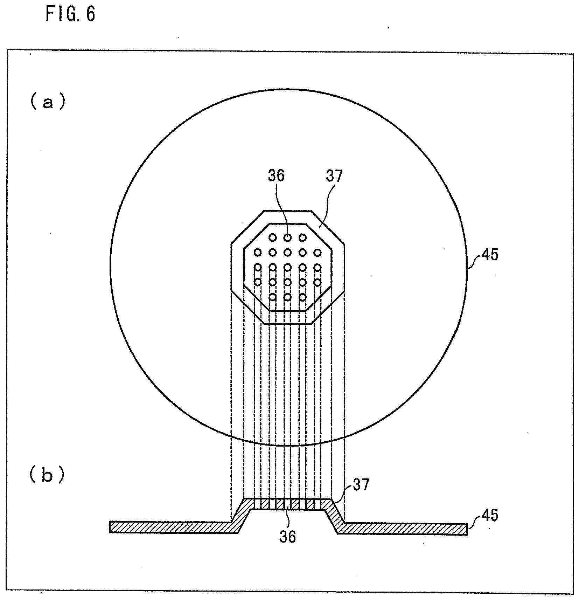

Fig. 6 illustrates a modified example of a vibrating plate according to this embodiment. Fig. 6 is a view schematically illustrating a vibrating plate 45, and (a) of Fig. 6 is a top view and (b) of Fig. 6 is a cross-sectional view.

The vibrating plate 45 is different from the vibrating plate 32 in the following point. That is, in the vibrating plate 32, the convex part 37 has a truncated-cone shape, whereas, in the vibrating plate 45, the convex part 37 has an octagonal frustum shape. In this embodiment, not only the vibrating plate 32 and/or the like but also the vibrating plate 45 can be used.

In Fig. 6, the plurality of micropores 36 are provided only in the upper base of the convex part 37 of the vibrating plate 45. However, the vibrating plate 45 may be structured as described above, in addition, may be structured in which the plurality of micropores 36 are provided not only in the upper base but also in a side surface section of the convex part 37, or are provided over the whole vibrating plate 45 including the side surface section. In the above description, the vibrating plate 45 has been described as a plate having an octagonal frustum shape. However, the vibrating plate 45 may have an n-gonal frustum shape such as a quadrangular frustum shape, a hexadecagonal frustum shape, or the like.

All the vibrating plate 32, the vibrating plate 40, and the vibrating plate 45 are the same in that (i) a cross section thereof in a direction in which the solution is to be atomized has a trapezoid shape and (ii) a surface having an atomizing opening of the solution is a plane surface. Note, however, that upper bases and side surfaces of the frustum-shaped vibrating plate 32, the vibrating plate 40, and the vibrating plate 45 do not need to be exactly plane, and may be a surface having a slight curvature.

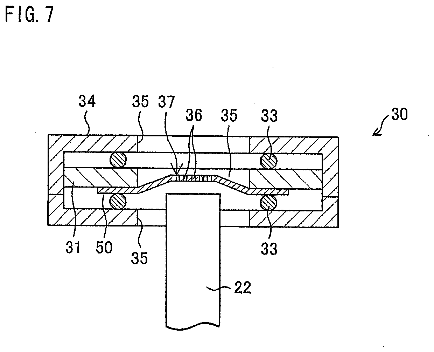

In the atomizing section 30 shown in Fig. 3, a rising section of the truncated-cone-shaped convex part 37 provided on the vibrating plate 32 is close to a center, however, as shown in Fig. 7, there may be used the vibrating plate 50 in which the rising section of the truncated-cone-shaped convex part 37 is close to an inner peripheral surface of the piezoelectric element 31.

### (Effect Confirmation Test 1)

The following description will discuss effects obtained by the ultrasonic atomizing device 1 with reference to results of Effect Confirmation Test 1.

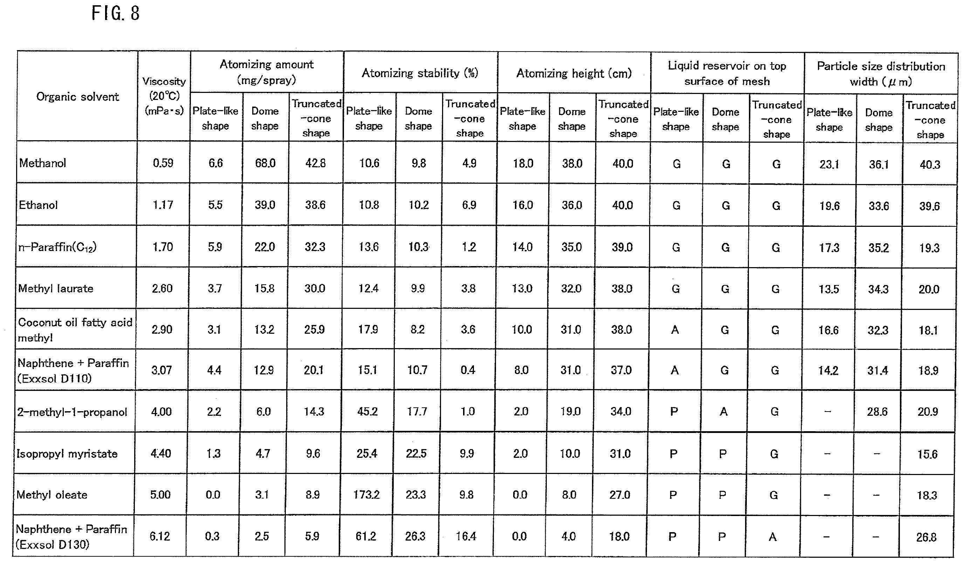

In Effect Confirmation Test 1, there were confirmed atomizing amounts etc. obtained when a plate-like vibrator (Comparative Example 1), a dome-shaped vibrator (Comparative Example 2), and a truncated-cone shaped vibrator (Example) were used with a plurality of organic solvents having a range of the viscosity of 0.59 mPa·s to 6.12 mPa·s (at 20 °C). The organic solvents for use in experiments are shown in Fig. 8. Fig. 8 is a table showing relationships between viscosity and atomizing amount etc. of solution, and, as shown in the drawing, 10 organic solvents from ethanol having viscosity of 0.59 mPa·s (at 20 °C) to naphthene + paraffin (Exxsol D130) having viscosity of 6.12 mPa·s (at 20 °C) were used.

As shown in Fig. 8, in Effect Confirmation Test 1, not only the atomizing amount (mg/spray), but also atomizing stability (%), atomizing height (cm), liquid reservoir on a top surface of the vibrating plate, and a particle size distribution width (µm) of each atomized solution were also measured.

Among them, the atomizing amount (mg/spray) indicates an amount of a solution for each atomization, and the larger the atomizing amount becomes, the more an effect obtained by atomization (sterilization, killing insects, etc.) increases.

The atomizing stability (%) indicates a standard deviation of stability (absence of variation) of the amount of the solution for each atomization, and the lower a numeral value is, the more excellent the atomizing stability is and the more stable an effect obtained by atomization is.

The atomizing height (cm) is obtained by visually observing an atomizing height, assuming that an atomizing opening of the solution in the vibrating plate is used as a reference point, and the higher a numeral value is, the higher the atomizing height is. This means that it is possible to scatter the solution in a wide range.

Regarding the liquid reservoir on the top surface of the vibrating plate, a case where the liquid reservoir does not occur on the top surface of the vibrating plate is denoted as G (Good), a case where the liquid reservoir occurs on the top surface of the vibrating plate but disappears within 1 second is denoted as A (Average), and a case where the liquid reservoir occurs and does not disappear within 1 second is denoted as P (Poor). In a case where liquid reservoir is generated, atomization of the solution is inhibited by the liquid reservoir, and therefore it is preferable that the liquid reservoir is not generated.

The particle size distribution width (µm) indicates a width of particle size distribution from D₁₀ to D₉₀, and the lower the particle size distribution width is, the more stable a particle size is. Stable particle size means that effects (sterilization, killing insects, etc.) can be stably obtained by atomization.

### [Ultrasonic wave atomizing device]

Experimental conditions common/unique to Comparative Example 1, Comparative Example 2, and Example are as follows.

### (Experimental conditions common to Comparative Example 1, Comparative Example 2, and Example)

(1) Piezoelectric vibrator: piezoelectric ceramics having an outer diameter of 15 mm, an inner diameter of 5 mm, and a thickness of 0.4 mm
(2) Applied voltage: 30 Vp-p
(3) Frequency of piezoelectric vibrator (ultrasonic exciter): 110 kHz
(4) Atomizing time: 1.0 second
(5) Diameter of micropores of vibrating plate: 10.0 µm
(6) Experiment temperature: 20 °C

### (Experimental conditions unique to each of Comparative Example 1, Comparative Example 2, and Example)

### (1) Comparative Example 1 (plate-like vibrating plate)

In Comparative Example 1, a liquid absorbent wick having a diameter of 4.5 mm suitable for the plate-like vibrating plate was used. The liquid absorbent wick has a porosity of 70% and has a pointed tip (on a vibrating plate side).

### (2) Comparative Example 2 (dome-shaped vibrating plate)

In Comparative Example 2, a liquid absorbent wick having a diameter of 4.5 mm suitable for the dome-shaped vibrating plate was used. The liquid absorbent wick has a porosity of 70%.

### (3) Example (truncated-cone-shaped vibrating plate)

In Example, a liquid absorbent wick having a diameter of 3.5 mm suitable for the truncated-cone shaped vibrating plate was used. The liquid absorbent wick has a porosity of 50% and is obtained by integrating a liquid absorbent wick and an absorber obtained by laminating two pieces of nonwoven fabric (produced by Asahi Kasei Corp., BEMCOT).

Results in each item, such as atomizing amount, obtained under the above experimental conditions are shown in Fig. 8, and the contents of each item will be described below.

### (1) Atomizing amount (mg/spray)

According to the experimental results in terms of atomizing amount, the atomizing amount of Comparative Example 1 (plate-like shape) is extremely lower than those of Comparative Example 2 (dome shape) and Example (truncated-cone shape) within a viscosity range in which the experiments were carried out.

In comparison of Comparative Example 2 with Example, the atomizing amount of ethanol having viscosity of 1.17 mPa·s (at 20 °C) was 39.0 (mg/spray) in Comparative Example 2 and 38.6 (mg/spray) in Example, which was not largely different. However, the atomizing amount of n-paraffin (C₁₂) having viscosity of 1.70 mPa·s (at 20 °C) was 22.0 (mg/spray) in Comparative Example 2 and 32.3 (mg/spray) in Example, i.e., the atomizing amount in Example was about 1.5 times as much as the atomizing amount in Comparative Example 2. Such tendency was similar from n-paraffin (C₁₂) having viscosity of 1.70 mPa·s (at 20 °C) to naphthene +paraffin (Exxsol D130) having viscosity of 6.12 mPa·s (at 20 °C).

The results show that, in a case where a solution has viscosity of 1.70 mPa·s (at 20 °C) or more, an atomizing amount of the solution is larger in a case of the truncated-cone-shaped vibrating plate than in a case of the plate-like and dome-shaped vibrating plates, and therefore it is possible to immediately atomize, into air, an active ingredient amount which satisfies effects of sterilization, killing insects, etc. enough, and the truncated-cone-shaped vibrating plate can exert greatest effects of sterilization, killing insects, etc. in a case where the devices are operated under a same condition. The results further show that, because the atomizing amount of the solution is large, it is possible to use a least active ingredient content in the solution, and therefore it is possible to reduce costs and to extremely improve the solution in safety against the case where the solution is touched.

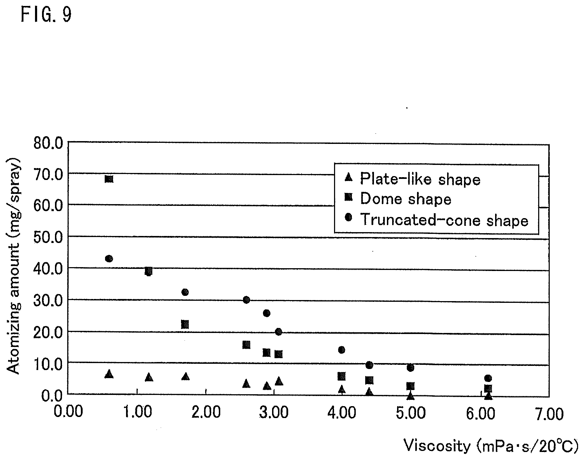

Fig. 9 is a view showing relationships between viscosity and atomizing amount when the plate-like, dome-shaped, and truncated-cone-shaped vibrating plates are used. The horizontal axis indicates viscosity (mPa·s (at 20 °C)) and the vertical axis indicates atomizing amount (mg/spray).

As shown in the drawing, in a case where the viscosity is 1.70 mPa·s (at 20 °C) or more, the truncated-cone-shaped vibrating plate produces the largest atomizing amount, the dome-shaped vibrating plate produces the second largest atomizing amount, and the plate-like vibrating plate produces the third largest atomizing amount. In a case of using the viscosity of 5 mPa·s (at 20 °C), the atomizing amount of the truncated-cone-shaped vibrating plate is about 2.9 times as much as that of the dome-shaped vibrating plate, which is a largest difference and, in a case of using the viscosity 1.70 mPa·s (at 20 °C), the atomizing amount of the truncated-cone-shaped vibrating plate is about 1.5 times as much as that of the dome-shaped vibrating plate, which is a smallest difference.

As a result, in particular in a case where the viscosity is 1.70 to 5.00 mPa·s (at 20 °C), the atomizing amount of the truncated-cone-shaped vibrating plate is larger than those of the plate-like-shaped and dome-shaped vibrating plates.

### (2) Atomizing stability (%)

According to the experimental results in terms of atomizing stability, the numeral value of Example (truncated-cone shape) is smaller than those of Comparative Example 1 (plate-like shape) and Comparative Example 2 (dome shape) within a whole viscosity range in which the experiments were carried out, and the vibrating plate in Example is excellent in atomizing stability. In particular, the atomizing stability is largely different within the range of viscosity of 1.70 to 5.00 mPa·s (at 20 °C). From this, the truncated-cone shaped vibrating plate is superior to the plate-like and dome-shaped vibrating plates in terms of atomizing stability and stably exerts the effects obtained by atomization. Note that, in a case where naphthene + paraffin (Exxsol D130) having viscosity of 6.12 mPa·s (at 20 °C) was atomized, the atomizing stability of the truncated-cone shaped vibrating plate became 16.4%, which was high.

From the results, the truncated-cone-shaped vibrating plate has higher atomizing stability than the plate-like and dome-shaped vibrating plates in a case where the viscosity is 1.70 mPa·s (at 20 °C) or more. Therefore, the truncated-cone-shaped vibrating plate can solve such a problem that the effects such as sterilization and killing insects cannot be satisfactorily obtained because an atomization operation having an extremely small atomizing amount is performed due to low atomizing stability. The truncated-cone-shaped vibrating plate can also solve such a problem that a guaranteed number of times of atomization of a solution bottle cannot be satisfied because an atomization operation having an extremely large atomizing amount is performed due to low atomizing stability.

### (3) Atomizing height (cm)

According to the experimental results in terms of atomizing height, Example (truncated-cone shape) has higher atomizing height than Comparative Example 1 (plate-like shape) and Comparative Example 2 (dome shape) within a whole viscosity range in which the experiments were carried out, and is superior to Comparative Example 1 (plate-like shape) and Comparative Example 2 (dome shape) in terms of diffusibility of a solution. In particular, the truncated-cone-shaped vibrating plate has an extremely higher atomizing height than the plate-like vibrating plate. In the viscosity range of 4.00 to 5.00 mPa·s (at 20 °C), the atomizing height of the dome-shaped vibrating plate is sharply declined, meanwhile, such a change in numeral value is not found in the truncated-cone-shaped vibrating plate. However, in a case where naphthene + paraffin (Exxsol D130) having viscosity of 6.12 mPa·s (at 20 °C) is atomized, even if the atomizing height of the truncated-cone shaped vibrating plate was 18.0 cm, which was low. This suggests that, when the viscosity becomes more than 5.00 mPa·s (at 20 °C), the viscosity is likely to affect the atomizing height.

### (4) Liquid reservoir on top surface of vibrating plate

According to the experimental results in terms of liquid reservoir on the top surface of the vibrating plate, only in a case where naphthene + paraffin (Exxsol D130) having viscosity of 6.12 mPa·s (at 20 °C) was atomized in Example (truncated-cone shape), liquid reservoir was found, however, there is no liquid reservoir in other viscosity ranges. Note that the liquid reservoir generated when the viscosity was 6.12 mPa·s (at 20 °C) disappears within 1 second.

Meanwhile, there is a tendency that the higher the viscosity in Comparative Example 1 (plate-like shape) and Comparative Example 2 (dome shape) is, the more easily liquid reservoir occurs, and the liquid reservoir was found in coconut oil fatty acid methyl having viscosity of 2.9 mPa·s (at 20 °C) in a case where the plate-like shape vibrating plate was used and the liquid reservoir was found in 2-methyl-1-propanol having viscosity of 4.00 mPa·s (at 20 °C) in a case where the dome-shaped vibrating plate was used.

As described above, in a case where liquid reservoir is generated, atomization of a solution is inhibited because of the liquid reservoir, and therefore it is preferable that a liquid reservoir is not generated. Also in terms of liquid reservoir, the truncated-cone-shaped vibrating plate has excellent atomizing stability.

Note that the dome-shaped vibrating plate has micropores over a whole surface of the dome portion, and therefore does not have a flat portion such as the upper base of the truncated-cone-shaped vibrating plate. Therefore, in the dome-shaped vibrating plate, a liquid tends to be leaked from micropores provided in a side surface, which does not atomize a solution, of the dome portion. Further, the dome-shaped vibrating plate tends to affect the atomizing stability unless the whole surface of the dome portion is in contact with the liquid absorbent wick. On the contrary, in a case of the truncated-cone-shaped vibrating plate, there is low possibility that liquid reservoir is generated in the micropores provided in portions other than the upper base, so that, also in this point, the truncated-cone-shaped vibrating plate has a structure in which liquid reservoir is less easily generated, as compared with the dome-shaped vibrating plate. Therefore, the truncated-cone-shaped vibrating plate has excellent atomizing stability.

### (5) Particle size distribution width (µm)

According to the experimental results in terms of particle size distribution width, in a case of methanol having viscosity of 0.59 mPa·s (at 20 °C) and ethanol having viscosity of 1.17 mPa·s (at 20 °C), particle size distribution widths in Example (truncated-cone-shape) are wider than those in Comparative Example 1 (plate-like shape) and Comparative Example 2 (dome shape).

However, within a viscosity range of n-paraffin (C₁₂) having viscosity 1.70 mPa·s (at 20 °C) or more, the particle size distribution width of the dome-shaped vibrating plate is the widest, meanwhile, the particle size distribution width of the truncated-cone-shaped vibrating plate is smaller. The particle size distribution width of the truncated-cone-shaped vibrating plate was not largely changed in a case where the viscosity is in a range of 1.70 mPa·s (at 20 °C) to 5.00 mPa·s (at 20 °C). Note, however, that, in a case of naphthene + paraffin (Exxsol D130) having the viscosity of 6.12 mPa·s (at 20 °C), the particle size distribution width of the truncated-cone-shaped vibrating plate is wider, i.e., 26.8 pm.

Note that, in a case where the viscosity was 4.00 or more in the plate-like vibrating plate and the viscosity was 4.40 or more in the dome-shaped vibrating plate, it was difficult to observe the particle size distribution width. From this, there is a tendency that in a case of the plate-like and dome-shaped vibrating plates, the particle size becomes unstable as the viscosity becomes higher.

### (6) Summary

As described above, the truncated-cone shaped vibrating plate is superior to the plate-like and dome-shaped vibrating plates in terms of all measurement items such as atomizing amount, atomizing stability, atomizing height, liquid reservoir on top surface of vibrating plate, and particle size distribution width of atomized solution. This superiority was remarkable in the viscosity range of 1.70 to 5.00 mPa·s (at 20 °C). Within the viscosity range of 1.7 mPa·s (at 20 °C) to 4.0 mPa·s (at 20 °C), an atomizing amount of a solution in the truncated-cone-shaped vibrating plate is further increased and the truncated-cone-shaped vibrating plate has satisfactory atomizing stability. The reason why such experimental results were obtained is as follows.

Specifically, in Example, the vibrating plate has a truncated cone shape, and, in a case where a solution having viscosity of 1.7 mPa·s (at 20 °C) to 5.0 mPa·s (at 20 °C) is particularly used, the truncated-cone-shaped vibrating plate can compensate for a problem that is likely to happen when a solution having low viscosity is used. In addition, it is possible to obtain a good point obtained when the solution having the low viscosity is used.

Specifically, in a case of a solution having low viscosity, an atomizing amount of the solution is generally increased, however, atomizing stability is lost in some cases. In view of this, in Example, atomization is stabilized by using the truncated-cone-shaped vibrating plate which does not easily generate liquid reservoir. Therefore, the truncated-cone-shaped vibrating plate can solve instability of atomization which is likely to happen in a case where a solution having low viscosity is used.

Further, a solution having low viscosity has such a good point that its atomizing amount generally tends to be large. In view of this, combination of a solution having low viscosity with the truncated-cone-shaped vibrating plate further improves the atomizing amount and the atomizing height and improves diffusibility of the solution, thereby improving the effects of the solution such as killing insects etc. The particle size distribution width is slightly changed as long as a solution having low viscosity of 1.7 mPa·s (at 20 °C) to 5.0 mPa·s (at 20 °C) is combined with the truncated-cone-shaped vibrating plate. This makes it possible to keep the atomizing stability and therefore stably obtain the effects (such as killing insects, sterilization, etc.).

As described above, Effect Confirmation Test 1 shows that, by combining a solution having low viscosity of 1.7 mPa·s (at 20 °C) to 5.0 mPa·s (at 20 °C) with a truncated-cone-shaped vibrating plate, it is expected that an effect of improving diffusibility of the solution or other effects will be obtained.

Note that various conditions such as a diameter of micropores in Effect Confirmation Test 1 do not limit a specification of the ultrasonic atomizing device 1. In Effect Confirmation Test 1, a truncated-cone vibrating plate was used, however, a truncated-pyramid vibrating plate is also expected to have effects similar to those of the truncated-cone-shaped vibrating plate because of its structure.

### (Effect Confirmation Test 2)

The following description will discuss an ultrasonic wave atomizing device according to this embodiment more specifically with reference to the following examples. Note, however, that an ultrasonic wave atomizing device according to this embodiment is not limited thereto.

### (Production of ultrasonic atomizing device)

An ultrasonic atomizing device having the following specifications was produced.
(1) Piezoelectric element 31: Piezoelectric ceramics whose outer diameter is 15 mm, inner diameter is 5 mm, and thickness is 0.4 mm
(2) Applied voltage: 30 Vp-p
(4) Frequency of piezoelectric element 31 (ultrasonic exciter): 110 kHz

### (Example)

As Example, the vibrating plate 32 and the vibrating plate 40 each made from nickel were used. The micropores 36 are provided only in the upper base of the vibrating plate 32, whereas the micropores 36 are provided over the whole vibrating plate 40. The micropores of both the vibrating plates 32 and 40 have a diameter of 6.0 µm, and sizes of the vibrating plates 32 and 40 are such that a diameter of the upper base of the convex part is 2.5 mm, a diameter of the lower base of the convex part is 5.0 mm, and a height of the convex part is 0.2 mm. Note that a solution used in this example was ethanol (viscosity: 1.17 mPa·s (20 °C)). This solution was also used in the following Comparative Example.

### (Comparative Example)

As Comparative Example, a vibrating plate A and a vibrating plate B each made from nickel and having a dome shape were used. Here, the "dome shape" means a shape of a vibrating plate whose convex part is expanded in a direction in which a solution is atomized so as to have an R shape. The vibrating plate A has micropores only in a surface constituting a dome. The vibrating plate B has micropores over its whole surface. The micropores of the vibrating plates A and B have a diameter of 6 µm, and sizes of the vibrating plates A and B are such that a diameter of a base end section of the convex part is 3 mm and a height of the convex part is 0.2 mm.

The solution was atomized under the above conditions in Example and in Comparative Example, and a height from an atomizing opening to an observable farthest destination point of a mist was measured visually. Note that an average value of results of 10 measurements was employed as a measurement value.

As a result, the farthest destination point of the mist in (Example) was 41.3 cm in a case of using the vibrating plate 32, and was 33.1 cm in a case of using the vibrating plate 40. Meanwhile, the farthest destination point of the mist in (Comparative Example) was 24.3 cm in a case of using the vibrating plate A and was 23.8 cm in a case of using the vibrating plate B.

That is, the results showed that the farthest destination point of the mist was higher in a case of the truncated-cone-shaped vibrating plate 32 and the truncated-cone-shaped vibrating plate 40 according to Example than in a case of the dome-shaped vibrating plate A and the dome-shaped vibrating plate B in Comparative Example. A comparison of the numerical values revealed that a solution atomizing height of the vibrating plate 32 was about 1.7 times as high as those of the vibrating plate A and the vibrating plate B, and a solution atomizing height of the vibrating plate 40 was about 1.4 times as high as those of the vibrating plate A and vibrating plate B.

From this above, Example demonstrated that diffusibility of the solution was improved by increasing the atomizing height and, as a result, effects (humidification effect, fragrance effect, insecticidal and sterilizing effect, etc.) of the solution were widely spread and brought about immediately, as compared with Comparative Example. Therefore, Example is effective particularly when the present invention is used as an insecticide or the like which needs to have an immediate effect.

Furthermore, Example had the inventors of the present invention find that durability of vibrating plates was improved.

The following description will discuss Example and Comparative Example specifically. The vibrating plates used in the above (Example) and (Comparative Example) were used and were driven under a driving condition that a device continuously performed idle driving at an interval of 2 seconds ON and 8 seconds OFF for 20 hours, and a degradation ratio of the vibrating plates were compared. Here, degradation of the vibrating plates indicates a case where 50% or more of an atomizing amount is reduced before and after this experiment. As a result of 9 experiments, in Example, none of the vibrating plate 32 was degraded. That is, the degradation ratio was 0%. Further, 2 vibrating plates 40 were degraded in 9 experiments. That is, the degradation ratio was 2/9 = 22%.

Meanwhile, in Comparative Example, 3 vibrating plates A and 3 vibrating plates B were degraded in 5 experiments. That is, each of the vibrating plates A and B had a degradation ratio of 60%.

The result showed that the degradation ratios of the truncated-cone-shaped vibrating plates according to Example were lower than those of dome-shaped vibrating plates according to Comparative Example. Particularly, the vibrating plate 32 having the micropores 36 only in the upper base has the degradation ratio of 0%, meanwhile, the degradation ratio was 60% in Comparative Example, so that the durability of the vibrating plate 32 is extremely high.

The truncated-cone-shaped convex part 37 of the vibrating plate 32 and a dome part of the vibrating plate A(B) are both formed by subjecting a plane vibration plate having micropores to a press process. In the vibrating plate A(B), when the plane vibration plate is pressed, process distortion occurs in the whole dome part, in particular, in a top portion of the dome part. On the contrary, process distortion rarely occurs in the upper base of the truncated-cone-shaped convex part 37 of the vibrating plate 32(40). Therefore, it is considered that a crack is generated by vibration at the time of driving more easily in the top part of the dome part than in the upper base of the truncated-cone-shaped convex part 37, and the degradation ratio of the vibrating plate is higher.

It is quite possible that a user unknowingly turns on the device in a state in which the solution container is not provided or the solution is exhausted (solution container becomes empty) while the device is being used, and, in such a case, if the degradation of the vibrating plate can be reduced, a life of the device can be prolonged. That is, by improving the durability of the device, it is possible to reduce a burden on a user also in terms of costs.

As described above, the results of Effect Confirmation Tests 1, 2 have been described. As described above, the truncated-cone-shaped vibrating plate is superior to the plate-like and dome-shaped vibrating plates in terms of all measurement items such as atomizing amount, atomizing stability, atomizing height, liquid reservoir on top surface of vibrating plate, and particle size distribution width of atomized solution. Thus, by using the truncated-cone-shaped vibrating plate, it is possible to improve diffusibility of a solution and effects (humidification effect, fragrance effect, insecticidal and sterilizing effect, etc.) of the solution are widely spread, and it is therefore expected that a high immediate effect will be obtained.

The degradation ratio of the truncated-cone-shaped vibrating plate can be remarkably reduced in comparison with conventional vibrating plates. Therefore, as long as the vibrating plates are made from a same material, it is possible to prolong the life of the device by using the truncated-cone-shaped vibrating plate, which results in reduction of a frequency of exchanging the device and a burden on a user is reduced in terms of costs.

### (Supplementary remarks)

In the ultrasonic wave atomizing device in accordance with the present invention, the solution may be a solution having the viscosity of 1.7 mPa·s (at 20 °C) to 4.0 mPa·s (at 20 °C).

In a case where the solution has the viscosity of 1.7 mPa·s (at 20 °C) to 4.0 mPa·s (at 20 °C), an atomizing amount of the solution can be further increased and atomizing stability can be satisfactorily kept.

In the ultrasonic wave atomizing device according to the present invention, the multiple micropores may be provided only in an upper base of the convex part of the vibrating plate.

According to the above structure, the ultrasonic wave atomizing device according to the present invention further improves an atomizing height of a liquid and durability of the vibrating plate.

In the ultrasonic wave atomizing device according to the present invention, the multiple micropores may be provided over a whole area of the piezoelectric vibrating plate.

According to the above structure, the ultrasonic wave atomizing device according to the present invention can further increase the durability of the vibrating plate, as compared with a conventional ultrasonic wave atomizing device including a dome-shaped vibrating plate.

In the ultrasonic wave atomizing device according to the present invention, the convex part of the vibrating plate may have a truncated-cone shape.

In the ultrasonic wave atomizing device according to the present invention, the convex part of the vibrating plate may have a truncated-pyramid shape.

In the ultrasonic wave atomizing device according to the present invention, the convex part of the vibrating plate may have a truncated-cone shape or a truncated-pyramid shape.

Therefore, in each device, in order to match the shape of the convex part of the vibrating plate with a layout of the device or a shape of the liquid absorbent wick, the shape may be changed to a truncated-cone shape or truncated-pyramid shape, so that a design of the device can be changed flexibly.

The ultrasonic wave atomizing device according to the present invention may be structured such that: the solution storage container is removably housed in the ultrasonic wave atomizing device; the solution storage container includes an absorber for supplying, to the vibrating plate, the liquid absorbed by the liquid absorbent wick; and the absorber is configured to be provided to or removed from the ultrasonic atomizing device together with the solution storage container when the solution storage container is provided to or removed from the ultrasonic atomizing device.

According to the above structure, in a case where the solution storage container is detached from the ultrasonic atomizing device, the absorber is detached together with the solution storage container from the device to an outside thereof, and does not remain in the ultrasonic atomizing device. Therefore, in a case where the solution in the solution storage container is exhausted and the absorber is dried, the absorber as well as the solution storage container is exchanged when the solution storage container is exchanged, so that, when the ultrasonic atomizing device is operated again, it is possible to prevent fibers etc. derived from the absorber from clogging micropores of the vibrating plate. This effect is exerted particularly when the vibrating plate is fixed to the side of the device body.

Therefore, the ultrasonic atomizing device according to the present invention can reduce the following situations: an atomizing amount of the solution is unstable because of this clogging; and a user is forced to exchange a high-cost vibrating plate.

As described above, because the ultrasonic wave atomizing device according to the present invention reduces clogging of the micropores of the vibrating plate, it is possible to improve atomizing stability of the ultrasonic wave atomizing device and it is also possible to reduce a burden on a user in terms of costs.

In the ultrasonic wave atomizing device according to the present invention, the truncated-cone-shaped convex part may have a rising section provided to be close to an inner peripheral surface of the piezoelectric element.

Various forms of the ultrasonic wave atomizing device according to this embodiment have been described above. Those forms are an example of this embodiment, and the forms described herein can be combined.

The present invention is not limited to the description of the embodiments above, and can be modified in numerous ways by a skilled person as long as such modification falls within the scope of the claims. An embodiment derived from a proper combination of technical means disclosed in different embodiments is also encompassed in the technical scope of the present invention.

### Industrial Applicability

The present invention is suitably used as an ultrasonic wave atomizing device for humidification, fragrance, sterilization and killing insects.

### Reference Signs List

- 1: ultrasonic wave atomizing device
- 10: device body
- 20: solution container (solution storage container)
- 21: container body
- 22: liquid absorbent wick
- 23: absorber
- 24: opening
- 30: atomizing section
- 31: piezoelectric element
- 32, 40, 45, 50: vibrating plate
- 33: elastic rings
- 34: casing
- 35: opening
- 36: micropores
- 37: convex part

## Claims

1. An ultrasonic wave atomizing device (1) for solution atomization, comprising:
a solution storage container (20) containing a solution in which at least an organic solvent and an active ingredient are mixed and whose viscosity is 1.7 mPa·s (at 20 °C) to 5.0 mPa·s (at 20 °C);
a liquid absorbent wick (22) for absorbing the solution from the solution storage container (20); and
a vibrating plate (32, 40, 45, 50) which has multiple micropores (36) penetrating the vibrating plate (32, 40, 45, 50) in a thickness direction and atomizes the solution by vibration of a piezoelectric vibrator (31) which generates ultrasonic vibration when supplied with electricity,
the vibrating plate (32, 40, 45, 50) having a convex part (37) which has a frustum shape and is supplied with the solution via the liquid absorbent wick (22).

2. The ultrasonic wave atomizing device (1) as set forth in claim 1, wherein the solution is a solution having the viscosity of 1.7 mPa s (at 20 °C) to 4.0 mPa s (at 20 °C).

3. The ultrasonic wave atomizing device (1) according to claim 1 or 2, wherein the multiple micropores (36) are provided only in an upper base of the convex part (37) of the vibrating plate (32, 40, 45, 50).

4. The ultrasonic wave atomizing device (1) according to claim 1 or 2, wherein the multiple micropores (36) are provided over a whole area of the piezoelectric vibrator (31).

5. The ultrasonic wave atomizing device (1) according to any one of claims 1 to 4, wherein the convex part (37) of the vibrating plate (32, 40, 45, 50) has a truncated-cone shape.

6. The ultrasonic wave atomizing device (1) according to any one of claims 1 to 4, wherein the convex part (37) of the vibrating plate (32, 40, 45, 50) has a truncated-pyramid shape.

7. The ultrasonic wave atomizing device (1) according to any one of claims 1 to 6, wherein:
the solution storage container (20) is removably provided to the ultrasonic wave atomizing device (1);
the solution storage container (20) includes an absorber (23) for supplying, to the vibrating plate (32, 40, 45, 50), the solution absorbed by the liquid absorbent wick (22); and
the absorber (23) is configured to be provided to or removed from the ultrasonic atomizing device (1) together with the solution storage container (20) when the solution storage container (20) is provided to or removed from the ultrasonic atomizing device (1).

8. Use of an ultrasonic wave atomizing device (1) according to any one of claims 1 to 7 for atomizing the solution in which at least the organic solvent and the active ingredient are mixed and whose viscosity is 1.7 mPa·s (at 20 °C) to 5.0 mPa·s (at 20 °C).

9. The use according to claim 8, wherein the solution is a solution having the viscosity of 1.7 mPa·s (at 20 °C) to 4.0 mPa·s (at 20 °C).

## Patentansprüche

1. Ein Ultraschallwellenzerstäuber (1) zur Lösungszerstäubung, umfassend:
einen Lösungsspeicherbehälter (20), enthaltend eine Lösung, in der mindestens ein organisches Lösungsmittel und ein Wirkstoff gemischt sind und deren Viskosität 1,7 mPa·s (bei 20°C) bis 5,0 mPa·s (bei 20°C) beträgt;
einen flüssigkeitsabsorbierenden Docht (22) zum Absorbieren der Lösung aus dem Lösungsspeicherbehälter (20); und
eine Vibrationsplatte (32, 40, 45, 50), welche eine Mehrzahl an Mikroporen (36) aufweist, die die Vibrationsplatte (32, 40, 45, 50) in einer Dickenrichtung durchdringen und die Lösung durch Vibration einer piezoelektrischen Vibrationseinrichtung (31), welche Ultraschallschwingungen erzeugt, wenn sie mit Elektrizität versorgt wird, zerstäuben,
wobei die Vibrationsplatte (32, 40, 45, 50) einen konvexen Teil (37) mit einer Kegelstumpfform aufweist und durch den flüssigkeitsabsorbierenden Docht (22) mit der Lösung versorgt wird.

2. Der Ultraschallwellenzerstäuber (1) wie in Anspruch 1 dargelegt, wobei die Lösung eine Lösung mit einer Viskosität von 1,7 mPa·s (bei 20 °C) bis 4,0 mPa·s (bei 20 °C) ist.

3. Der Ultraschallwellenzerstäuber (1) gemäß Anspruch 1 oder 2, wobei die Mehrzahl an Mikroporen (36) nur in einer oberen Basis des konvexen Teils (37) der Vibrationsplatte (32, 40, 45, 50) bereitgestellt sind.

4. Der Ultraschallwellenzerstäuber (1) gemäß Anspruch 1 oder 2, wobei die Mehrzahl an Mikroporen (36) auf der ganzen Fläche der piezoelektrischen Vibrationseinrichtung (31) bereitgestellt sind.

5. Der Ultraschallwellenzerstäuber (1) gemäß einem der Ansprüche 1 bis 4, wobei der konvexen Teil (37) der Vibrationsplatte (32, 40, 45, 50) eine Kegelstumpfform aufweist.

6. Der Ultraschallwellenzerstäuber (1) gemäß einem der Ansprüche 1 bis 4, wobei der konvexe Teil (37) der Vibrationsplatte (32, 40, 45, 50) eine abgestumpfte Pyramidenform aufweist.

7. Der Ultraschallwellenzerstäuber (1) gemäß einem der Ansprüche 1 bis 6, wobei:
der Lösungsspeicherbehälter (20) abnehmbar in dem Ultraschallwellenzerstäuber (1) bereitgestellt ist;
der Lösungsspeicherbehälter (20) einen Absorber (23) beinhaltet, um der Vibrationsplatte (32, 40, 45, 50) die vom flüssigkeitsabsorbierenden Docht (22) absorbierte Lösung zuzuführen; und
der Absorber (23) so konfiguriert ist, dass er zusammen mit dem Lösungsspeicherbehälter (20) dem Ultraschallwellenzerstäuber (1) zugeführt oder von ihm entfernt wird, wenn der Lösungsspeicherbehälter (20) dem Ultraschallwellenzerstäuber (1) zugeführt oder von ihm entfernt wird.

8. Verwendung eines Ultraschallwellenzerstäubers (1) gemäß einem der Ansprüche 1 bis 7 zum Zerstäuben der Lösung, in der mindestens das organische Lösungsmittel und der Wirkstoff gemischt sind und deren Viskosität 1,7 mPa·s (bei 20 °C) bis 5,0 mPa·s (bei 20 °C) beträgt.

9. Die Verwendung gemäß Anspruch 8, wobei die Lösung eine Lösung mit einer Viskosität von 1,7 mPa·s (bei 20°C) bis 4,0 mPa·s (bei 20°C) ist.

## Revendications

1. Dispositif d'atomisation à ondes ultrasoniques (1) pour atomiser une solution, comprenant:
un récipient de stockage de solution (20) contenant une solution dans laquelle au moins un solvant organique et un ingrédient actif sont mélangés et dont la viscosité est de 1,7 mPa·s (à 20°C) à 5,0 mPa·s (à 20°C);
une mèche absorbant un liquide (22) pour absorber la solution à partir du récipient de stockage de solution (20); et
une plaque vibrante (32, 40, 45, 50) qui a de multiples micropores (36) pénétrant la plaque vibrante (32, 40, 45, 50) dans le sens de l'épaisseur et atomise la solution par vibration d'un vibrateur piézoélectrique (31) qui génère une vibration ultrasonique lorsqu'il est alimenté en électricité,
la plaque vibrante (32, 40, 45, 50) ayant une partie convexe (37) qui a la forme d'un tronc et est alimentée en la solution par l'intermédiaire de la mèche absorbant un liquide (22).

2. Dispositif d'atomisation à ondes ultrasoniques (1) selon la revendication 1, dans lequel la solution est une solution ayant une viscosité de 1,7 mPa·s (à 20°C) à 4,0 mPa·s (à 20°C).

3. Dispositif d'atomisation à ondes ultrasoniques (1) selon la revendication 1 ou 2, dans lequel les micropores multiples (36) sont fournis seulement dans une base supérieure de la partie convexe (37) de la plaque vibrante (32, 40, 45, 50).

4. Dispositif d'atomisation à ondes ultrasoniques (1) selon la revendication 1 ou 2, dans lequel les micropores multiples (36) sont fournis sur la totalité d'une aire du vibrateur piézoélectrique (31).

5. Dispositif d'atomisation à ondes ultrasoniques (1) selon l'une quelconque des revendications 1 à 4, dans lequel la partie convexe (37) de la plaque vibrante (32, 40, 45, 50) a la forme d'un cône tronqué.

6. Dispositif d'atomisation à ondes ultrasoniques (1) selon l'une quelconque des revendications 1 à 4, dans lequel la partie convexe (37) de la plaque vibrante (32, 40, 45, 50) a la forme d'une pyramide tronquée.

7. Dispositif d'atomisation à ondes ultrasoniques (1) selon l'une quelconque des revendications 1 à 6, dans lequel:
le récipient de stockage de solution (20) est fourni de façon amovible au dispositif d'atomisation à ondes ultrasoniques (1);
le récipient de stockage de solution (20) inclut un absorbeur (23) pour fournir, à la plaque vibrante (32, 40, 45, 50), la solution absorbée par la mèche absorbant un liquide (22); et
l'absorbeur (23) est configuré pour être fourni à ou enlevé du dispositif d'atomisation ultrasonique (1) conjointement avec le récipient de stockage de solution (20) lorsque le récipient de stockage de solution (20) est fourni à ou enlevé du dispositif d'atomisation ultrasonique (1).

8. Utilisation d'un dispositif d'atomisation à ondes ultrasoniques (1) selon l'une quelconque des revendications 1 à 7 pour atomiser la solution dans laquelle au moins le solvant organique et l'ingrédient actif sont mélangés et dont la viscosité est de 1,7 mPa·s (à 20°C) à 5,0 mPa s (à 20°C).

9. Utilisation selon la revendication 8, dans laquelle la solution est une solution ayant une viscosité de 1,7 mPa·s (à 20°C) à 4,0 mPa·s (à 20°C).
